# EUROPEAN PATENT APPLICATION

(11) **EP 4 497 744 A1**
(43) Date of publication of application: **29.01.2025**
(21) Application number: 23774944.5
(22) Date of filing: 22.03.2023
(51) Int. Cl.: C07C 67/08, C07B 61/00, C07C 69/88

(54) **METHOD FOR PRODUCING SALICYLIC ACID ESTER**

(30) Priority: 23.03.2022 JP 2022047108
(71) Applicant: API Corporation, Chikujo-gun, Fukuoka 871-0801 (JP)
(72) Inventor: KOBA, Yurie, Chikujo-gun, Fukuoka 871-0801 (JP); TANIIKE, Hirotsugu, Chikujo-gun, Fukuoka 871-0801 (JP); MURAI, Masato, Chikujo-gun, Fukuoka 871-0801 (JP)
(74) Representative: Godemeyer Blum Lenze Patentanwälte Partnerschaft mbB - werkpatent
(86) International application number: PCT/JP2023/011200
(87) International publication number: WO 2023/182349

(57) **Abstract**

A method for producing a salicylic acid ester represented by general formula (2) below, in which salicylic acid and an alcohol represented by general formula (1) below are brought into contact with a solid acid catalyst at a reaction temperature of 50°C to 200°C.

X-R-OH ... (1)

(In the formula (1), R represents a linear or branched alkyl group having 1 to 6 carbon atoms. X represents a hydrogen atom or a hydroxyl group.) 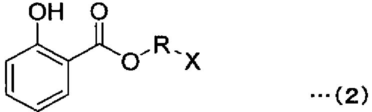 (In the formula (2), R and X are each as defined in the formula (1).)

## Description

### [Technical Field]

The present invention relates to a method for producing a salicylic acid ester that is useful as pharmaceuticals.

### [Background Art]

Methyl salicylate and glycol salicylate, which are salicylic acid esters, have been thus far in wide use as antiinflammatory analgesics.

Conventionally, as a method for producing methyl salicylate, a method in which methyl salicylate is produced with a batch-type reactor is known (NPL 1). For example, a method in which salicylic acid, methanol and concentrated sulfuric acid are added to a batch-type reactor and an esterification reaction is caused at a high temperature, thereby producing methyl salicylate as shown in the following reaction formula is known.

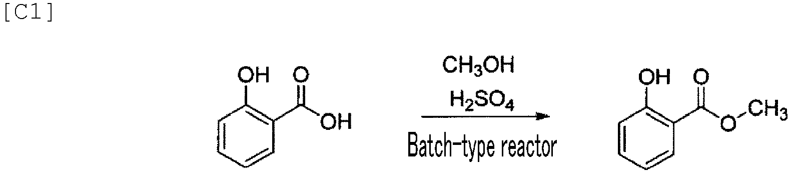

In addition, as a method for producing glycol salicylate, a method in which glycol salicylate is produced using a batch-type reactor is known (PTL 1). For example, a method in which salicylic acid, ethylene glycol and concentrated sulfuric acid are added to a batch-type reactor and an esterification reaction is caused at a high temperature, thereby producing glycol salicylate as shown in the following reaction formula is known.

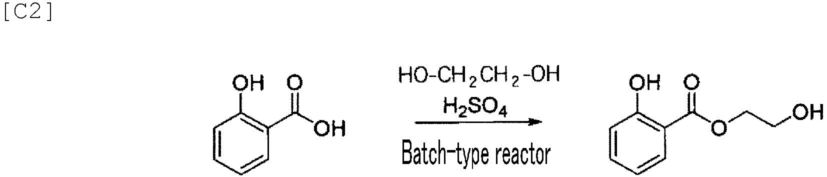

However, in the methods of NPL 1 and PTL 1, since concentrated sulfuric acid is used as a catalyst, and a large amount of alkali water is used during a neutralization treatment of concentrated sulfuric acid and unreacted salicylic acid in a reaction solution, a large amount of an acid waste liquid is generated, and a lot of time, expense and effort are required for the separation of the acid waste liquid from a target or an exhaust acid treatment. In this way, 15 million tons or more of sulfuric acid has been consumed as "unrecyclable catalysts" every year, and the waste of a large amount of energy and the discharge of waste have placed a large burden on the environment (PTL 4).

In addition, since sulfuric acid is highly toxic and corrosive, time, expense and effort taken to secure safety and maintain plants cannot be ignored.

Due to these facts, there has been a desire for replacing conventional acid catalyst reaction processes, which rely on sulfuric acid, by a highly efficient production method that places as little burden as possible on the environment.

Ordinarily, it is known that esterification reactions are reversible reactions. In esterification reactions, since an ester that is generated after the reaction and water cause a reverse reaction, there is a need to bias the equilibrium of the esterification reaction toward the generation system to obtain the ester, i.e., a target, at a high yield. As a method for biasing the equilibrium of the esterification reaction toward the generation system, normally, a method in which one reagent from either an alcohol or a carboxylic acid is used in excess or used to remove water and drive the water out of the system is known.

In a case where an esterification reaction is performed with a batch-type reactor, it is difficult to remove water that is generated in the system in many cases, and a lot of time and energy are required for the reaction.

In the method of PTL 1, as is clear from Comparative Example 1 to be described below, since the reaction time is long, a lot of impurities represented by the following formulae (a) and (b) (hereinafter, a compound represented by the formula (a) will be referred to as SEE, and a compound represented by the formula (b) will be referred to as DSE in some cases) are generated, and the purification load is high.

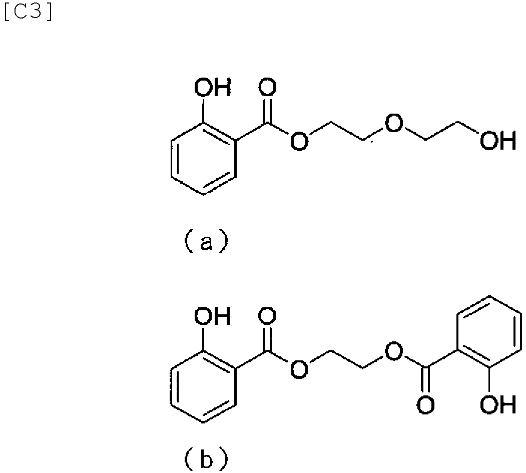

Therefore, there is a desire for the development of a safer and industrially advantageous high-productivity method for producing a salicylic acid ester, in which a salicylic acid ester can be generated within a short reaction time at a high selectivity and a high yield, and the environmental load is low, and moreover the amount of an impurity generated is small. Here, productivity means the relative proportion of a product that is obtained by the injection of production factors (a facility, land, a building, energy, a raw material, labor, time and the like) into an ordinary generation activity.

Esterification reactions for which an acid catalyst is used are divided into a case where the acid catalyst is melted in a reaction fluid and used (homogeneous system) and a case where the acid catalyst is present as a solid in the reaction fluid (heterogeneous system). Furthermore, the heterogeneous system is classified into a fluidized bed in which the acid catalyst flows together with a reaction fluid and a fixed bed in which the acid catalyst is at rest and does not move. It is known that, in esterification reactions using the fixed bed, the catalyst concentration per unit space can be set to be higher than that in esterification reactions using a fluidized bed or the homogeneous system, and it is possible to easily separate the reaction fluid and the catalyst. For example, production methods in which an esterification reaction is continuously performed using a fixed bed to enhance the productivity of the esterification reaction are known (PTL 2 and PTL 3).

However, in PTL 2 and 3, there are no descriptions of production methods in which an esterification reaction is continuously performed using a fixed bed as a method for producing a salicylic acid ester such as methyl salicylate and glycol salicylate.

### [Citation List]

### [Patent Literature]

[PTL 1] Description of West German Patent No. 164128
[PTL 2] Japanese Patent Application Publication No. 2019-172665
[PTL 3] Japanese Patent Application Publication No. H10-279523 [PTL 4] WO 2007/032188

### [Non Patent Literature]

[NPL 1] "Chemistry and Education", written by Akira Shimada, Vol. 59, Issue 8 (2011), pp. 422 to 425

### [Summary of Invention]

### [Technical Problem]

An object of the present invention is to provide a safer and industrially advantageous high-productivity method for producing a salicylic acid ester, in which a salicylic acid ester can be generated within a short reaction time at a high selectivity and a high yield, and the environmental load is low, and moreover the amount of an impurity generated is small. In the present specification, "selectivity" means the proportion of a salicylic acid ester generated in a plurality of products, and for "productivity", the yield of a target that is obtained per unit time is used as an index.

The present inventors found that, when salicylic acid and an alcohol are continuously passed through a column filled with a solid acid catalyst and reacted together, a salicylic acid ester can be generated within a short reaction time at a high selectivity and a favorable yield. The method is a safer-than-ever and industrially advantageous production method in which the amount of an impurity generated is small, and an environmental load is low, and moreover a salicylic acid ester can be continuously obtained at a high productivity and a low cost.

The features of the present invention are as described below.
[1] A method for producing a salicylic acid ester represented by general formula (2) below, in which salicylic acid and an alcohol represented by general formula (1) below are brought into contact with a solid acid catalyst at a reaction temperature of 50°C to 200°C.

   X-R-OH ... (1)

   (In the formula (1), R represents a linear or branched alkyl group having 1 to 6 carbon atoms. X represents a hydrogen atom or a hydroxyl group.) 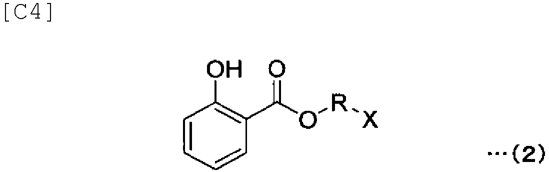 (In the formula (2), R and X are each as defined in the formula (1).)
[2] The method for producing a salicylic acid ester according to [1], in which the contact method is a method in which the salicylic acid and the alcohol are passed through a column filled with the solid acid catalyst.
[3] The method for producing a salicylic acid ester according to [1] or [2], in which the solid acid catalyst contains an organic adsorbent or an inorganic adsorbent as a base and has a strong acidic ion exchange group.
[4] The method for producing a salicylic acid ester according to any one of [1] to [3], in which the solid acid catalyst has an average particle diameter of 1 µm to 2000 µm.
[5] The method for producing a salicylic acid ester according to any one of [1] to [4], in which the solid acid catalyst has a specific surface area of 200 m²/g to 2000 m²/g.
[6] The method for producing a salicylic acid ester according to any one of [1] to [5], in which the reaction temperature is 70°C to 180°C.

### [Advantageous Effects of Invention]

### [Brief Description of Drawings]

[Fig. 1]

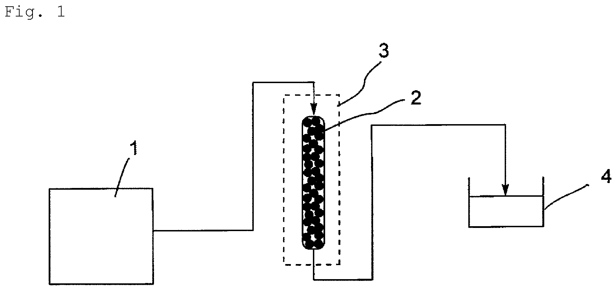

   Fig. 1 is a system diagram of a flow synthesis system showing one example of an embodiment of a method for producing a salicylic acid ester of the present invention.
[Fig. 2]

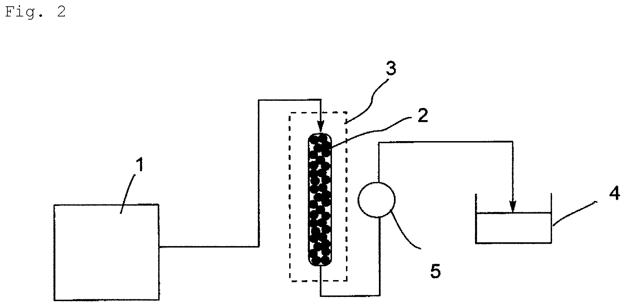

   Fig. 2 is a system diagram of a flow synthesis system including a back pressure valve showing another example of the embodiment of the method for producing a salicylic acid ester of the present invention.

### [Description of Embodiments]

Hereinafter, the present invention will be described in detail.

### [Method for producing salicylic acid ester]

A method for producing a salicylic acid ester of the present invention is a method in which a solution (hereinafter, referred to as the salicylic acid solution in some cases) of salicylic acid and an alcohol (hereinafter, referred to as "alcohol (1)" in some cases) represented by general formula (1) below is brought into contact with a solid acid catalyst. Preferably, the solution of salicylic acid and an alcohol is continuously passed through the column filled with the solid acid catalyst, whereby an esterification reaction (hereinafter, referred to as "the esterification reaction of the present invention" in some cases)is caused, and a salicylic acid ester (hereinafter, referred to as "salicylic acid ester (2)" in some cases) represented by general formula (2) below is continuously produced (hereinafter, this step will be referred to as "the esterification step of the present invention" in some cases).

X-R-OH ... (1)

(In the formula (1), R represents a linear or branched alkyl group having 1 to 6 carbon atoms. X represents a hydrogen atom or a hydroxyl group.) 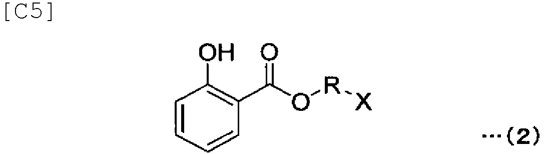 (In the formula (2), R and X are each as defined in the formula (1).)

Specific examples of the linear or branched alkyl group having 1 to 6 carbon atoms as R in the formulae (1) and (2) include lower alkyl groups such as a methyl group, an ethyl group, a linear or branched propyl group, a linear or branched butyl group, a linear or branched pentyl group and a linear or branched hexyl group.

In a case where X is a hydrogen atom, the alcohol (1) is a monovalent alcohol, and in a case where X is a hydroxyl group, the alcohol (1) is a divalent alcohol.

### <Esterification step>

The esterification step of the present invention means a step of causing an esterification reaction between salicylic acid and the alcohol to obtain an ester. A method for performing the esterification step is not particularly limited.

Examples thereof include a method by a flow synthesis system in which a salicylic acid solution prepared in a preparation tank 1 is continuously passed through a reaction container 3 including a column filled with a solid acid catalyst 2 of the present invention, an esterification reaction of salicylic acid with the alcohol is continuously performed in the presence of the solid acid catalyst in the column, and a liquid reaction product containing a salicylic acid ester that flows out from the column is received in a collection tank 4 as shown in Figs. 1 and 2.

The flow synthesis system of Fig. 2 is different from the flow synthesis system of Fig. 1 in that a back pressure valve 5 is provided in a flow path that feeds the liquid reaction product from the reaction container 3 to the collection tank 4, but otherwise has the same configuration.

This flow synthesis system will be described below.

### <Salicylic acid solution>

As the salicylic acid that is a raw material for producing the salicylic acid ester (2), a commercially available product may be used or an acid obtained by applying a well-known method may be used.

As the alcohol (1), normally, an alcohol corresponding to the salicylic acid ester (2), which is the target, is used. For example, methanol is preferably used in the case of producing methyl salicylate, and ethylene glycol is preferably used in the case of producing ethylene glycol salicylate.

The number of carbon atoms in R in the formula (1) is normally 1 to 6, preferably 1 to 4, and particularly preferably 1 or 2. R is a linear or branched alkyl group and preferably a linear alkyl.

The amount of the alcohol (1) used needs to be an amount at which the esterification reaction of the present invention progresses and is not particularly limited, but is normally 1 g to 100 g and preferably 3 g to 20 g per 1 g of the salicylic acid from the viewpoint of the productivity.

As a solvent that is used in the salicylic acid solution, a solvent that is capable of dissolving the salicylic acid and does not hinder the progress of the reaction can be used. As the solvent that is used in the present invention, normally, the alcohol (1) enabling the intended salicylic acid ester (2) to be obtained by the esterification reaction is preferably used. For example, from the viewpoint of suppressing the generation of an impurity, the alcohol (1) is preferably methanol in the case of producing methyl salicylate, and the alcohol (1) is preferably ethylene glycol in the case of producing glycol salicylate.

One of these solvents may be used singly or two or more thereof may be mixed and used in an arbitrary composition and an arbitrary ratio. The solvent is preferably used singly from the viewpoint of easiness in the removal of the solvent and the suppression of an impurity.

The concentration of the salicylic acid in the salicylic acid solution is not particularly limited as long as there is no hindrance in the circulation into the column. The concentration of the salicylic acid in the salicylic acid solution is normally 0.1 mass% to 80 mass%, preferably 5 mass% to 75 mass%, and particularly preferably 10 mass% to 70 mass% from the viewpoint of the productivity and the reactivity. In a case where the concentration of the salicylic acid is too low, there is a concern that the reaction time may become long, and in a case where the concentration is too high, there is a concern that the reaction may not sufficiently progress.

### <Solid acid catalyst>

As the solid acid catalyst, a catalyst that makes the esterification reaction between the salicylic acid and the alcohol (1) progress is used.

The solid acid catalyst in the present invention is normally an acid catalyst of a solid containing a base and an ion exchange group.

Examples of the base include an organic adsorbent and an inorganic adsorbent.

Examples of the organic adsorbent include aromatic adsorbents such as styrene/divinylbenzene copolymers and divinylbenzene polymers; acrylic (co)polymers such as methyl methacrylate/bis(methacrylic acid)ethylene glycol copolymers. From the viewpoint of the reactivity and the durability of the solid acid catalyst, the aromatic adsorbents are preferable, and the styrene/divinylbenzene copolymers are particularly preferable.

In a case where an organic adsorbent is used as the base of the solid acid catalyst, the degree of crosslinking of the organic adsorbent is normally 1 mass% to 20 mass% and preferably 2 mass% to 10 mass%. The degree of crosslinking represents the content (mass%) of divinyl benzene, which is a crosslinking agent, in the case of the styrene/divinylbenzene copolymers. When the degree of crosslinking is within the above range, it is possible to improve the reactivity and the durability of the solid acid catalyst.

Examples of the inorganic adsorbent include silica gel and alumina. Silica gel is preferable from the viewpoint of the cost and the reactivity.

As the ion exchange group in the solid acid catalyst, normally, strong acid ion exchange groups or weak acid ion exchange groups can be used. From the viewpoint of promoting the esterification reaction between salicylic acid and the alcohol (1), strong acid ion exchange groups are preferable, and among them, a sulfonic acid group or an alkylsulfonic acid group are particularly preferable.

The structure of the solid acid catalyst is not particularly limited, and gel-type, porous, highly porous or macroporous solid acid catalysts can be used. The shape of the solid acid catalyst is not particularly limited, and particulate, pellet-like, film-like or columnar solid acid catalysts can be used. From the viewpoint of the filling property into the column, particulate or pellet-like solid acid catalysts are preferable, and a particulate solid acid catalyst is more preferable.

As shown in Figs. 1 and 2, the solid acid catalyst is normally used in a state of filling the column.

The size of the solid acid catalyst is not particularly limited as long as the solid acid catalyst is capable of filling the column and catalyzing the reaction.

In a case where the shape of the solid acid catalyst is particulate, the particle diameter of the solid acid catalyst is normally 1 µm to 2000 µm and preferably 4 µm to 1000 µm from the viewpoint of the industrial handleability or the like.

The most frequent particle diameter of the solid acid catalyst is normally 50 µm to 2000 µm, preferably 150 µm to 1500 µm and particularly preferably 250 µm to 1000 µm.

Here, the particle diameter of the solid acid catalyst is the average particle diameter measured according to a normal method by the laser diffraction-type particle size distribution measurement method.

The specific surface area of the solid acid catalyst that is used in the present invention is preferably 100 m²/g to 2000 m²/g. The specific surface area is preferably 200 m²/g to 1000 m²/g from the viewpoint of the reactivity. The use of the solid acid catalyst having a specific surface area within the above range makes it possible to efficiently perform the reaction and to enhance the productivity.

As the solid acid catalyst that is used in the present invention, any solid acid catalysts can be used as long as the above-described conditions are satisfied, and examples thereof include commercially available products such as DIAION (registered trademark) SK104H, SK1BH, UBK530K, RCP145H manufactured by Mitsubishi Chemical Corporation; Amberlite (registered trademark) XH2071H manufactured by Organo Corporation; and TAYCACURE (registered trademark) SAC-6 manufactured by Tayca Corporation. Among these, SK104H, SK1BH and UBK530K are preferable from the viewpoint of the reactivity and the productivity.

The details of these commercially available solid acid catalysts will be shown in Table 1 below.

**[Table 1]**

| | Product name | Ion exchange group | Base |
|---|---|---|---|
| Solid acid catalyst 1 | DIAION^{™} SK104H | Sulfonic acid group | Styrene-DVB |
| Solid acid catalyst 2 | DIAION^{™} SK1BH | Sulfonic acid group | Styrene-DVB |
| Solid acid catalyst 3 | DIAION^{™} UBK530K | Sulfonic acid group | Styrene-DVB |
| Solid acid catalyst 4 | DIAION^{™} RCP145H | Sulfonic acid group | Styrene-DVB |
| Solid acid catalyst 5 | Amberlite^{®} XH2071H | Sulfonic acid group | Styrene-DVB |
| Solid acid catalyst 6 | TAYCACURE^{™} SAC-6 | Propyl sulfonic acid group | Silica gel |

In Table 1, "Styrene-DVB" is the "styrene/divinylbenzene copolymer".

One of these solid acid catalysts may be used singly or two or more thereof may be mixed and used.

### <Flow synthesis system>

A flow synthesis system suitable for performing the method for producing a salicylic acid ester of the present invention is a system in which a reaction container having an inlet and an outlet is used and "the injection of a raw material from the inlet", "the reaction" and "the collection of a product from the outlet" are performed at the same time. This concept is well known to persons skilled in the art (for example, "Flow Micro Synthesis" (Kagakudojin) published in 2014, p. 9).

In the flow synthesis system of the present invention, the shape of the column that is filled with the solid acid catalyst is not particularly limited as long as the column can be filled with the solid acid catalyst and is suitable for the reaction; however, normally, tubular columns can be used.

The material of the column relating to the present invention is not particularly limited. Examples of the material of the column include glass, stainless steel (SUS), Hastelloy and TEFLON (registered trademark), and stainless steel or Hastelloy is preferable.

The size of the column is not particularly limited as long as the column is suitable for the reaction. As the column, for example, thin tubular columns such as a column that is 10 mm in diameter and 100 mm in length and a column that is 10 mm in diameter and 250 mm in length can be used.

Examples of the column filled with the catalyst include 4.6 mm × 100 mm SUS columns or Hastelloy columns closely packed with the solid acid catalyst.

A tube that is used as a flow path for introducing the salicylic acid solution containing the salicylic acid and the alcohol (1), which are reactants, into the column and a flow path for discharging the liquid reaction product from the column is not particularly limited. Specific examples of the tube include TEFLON (registered trademark) tubes having an inner diameter of 1 mm.

The introduction of the salicylic acid solution containing the salicylic acid and the alcohol (1), which are the reactants, into the column and the discharge of the liquid reaction product can be performed by liquid delivery using a syringe pump, a diaphragm pump, a mass controller or the like.

A back pressure valve or an inline analyzer may be provided in the flow path on the side where the liquid reaction product is discharged from the column.

### <Reaction conditions>

The reaction temperature of the esterification reaction of the present invention means the outside temperature of the column filled with the solid acid catalyst of the present invention. From the viewpoint of the reactivity, the productivity and the like, the upper limit of the reaction temperature is normally 50°C or higher, preferably 70°C or higher, more preferably 90°C or higher, still more preferably 100°C or higher and particularly preferably 105°C or higher, and the lower limit is normally 200°C or lower, preferably 180°C or lower, more preferably 160°C or lower, still more preferably 150°C or lower and particularly preferably 140°C or lower.

In a case where the reaction temperature is lower than the above lower limit, there are cases where the reactivity deteriorates. In a case where the reaction temperature is higher than the above upper limit, there are cases where decreases in the yield and the purity or the deterioration of the solid acid catalyst due to a side reaction occurs.

The lower limit of the reaction pressure of the esterification reaction of the present invention is normally 0.1 MPa or higher, preferably 0.2 MPa or higher and particularly preferably 0.3 MPa or higher, and the upper limit is normally 1 MPa or lower, preferably 0.8 MPa or lower and particularly preferably 0.6 MPa or lower. When the esterification reaction is performed at a reaction pressure within the above range, it is possible to efficiently perform the reaction with the homogeneous system even at a temperature that is equal to or higher than the boiling point of the solvent.

The reaction pressure can be adjusted by applying a back pressure to the flow path posterior to the column filled with the solid acid catalyst of the present invention using the back pressure valve or the like. In the case of setting the reaction temperature to be equal to or higher than the boiling point of the solvent to be used, the reaction can be performed at a pressure adjusted so that the solvent becomes liquid even at the desired reaction temperature. The reaction pressure is not particularly limited, but is normally 0.1 MPa to 1 MPa.

The reaction time of the esterification reaction of the present invention means the time (residence time) during which a reaction solution resides in the column filled with the solid acid catalyst. The reaction time may vary with the reaction temperature or the reaction pressure, but is normally one minute to 120 minutes.

In the case of producing ethylene glycol salicylate, it is preferable to suppress the amount of a by-product generated with the above-described reaction conditions since SEE and DSE, which are by-products, are difficult to remove by a purification operation such as distillation and the purification load is high.

### <Post treatment>

The salicylic acid ester (2), which is the target, may be isolated from the liquid reaction product obtained by the esterification step of the present invention by a treatment, such as neutralization, separation, concentration or filtration, of this liquid reaction product or by well-known purification means such as crystallization or column chromatography.

### Examples

Hereinafter, the present invention will be described in more detail with examples. The scope of the present invention is not limited to the following examples.

### [Abbreviations]

In the examples, abbreviations represent the following compounds, respectively.
SA: Salicylic acid
SE: Ethylene glycol salicylate
SM: Methyl salicylate
SEE: Ethylene glycol salicylic acid ether
DSE: Ethylene glycol disalicylic acid ether
SEE and DSE are by-products.

The structural formulae of the individual compounds are as shown below.

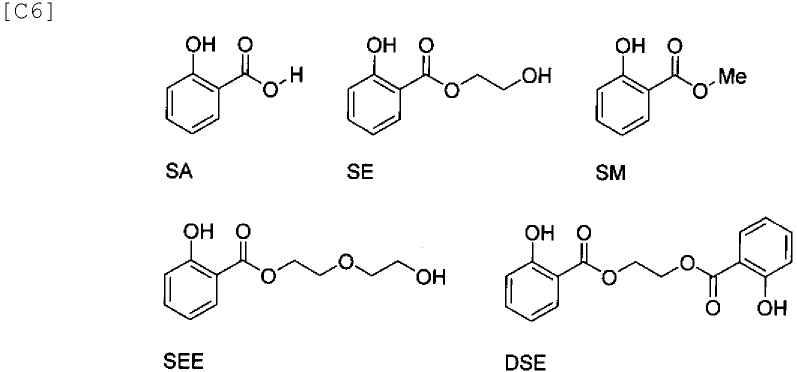

### [Flow synthesis apparatus]

In the following examples and comparative examples, the following flow synthesis apparatus was used.

### "MCR-1000" manufactured by EYELA

### [Analysis method 1 (HPLC)]

Apparatuses and conditions used for the analyses of liquid reaction products in the following examples and comparative examples are as described below.

### <Apparatuses and conditions>

Device: Agilent 1290 infinity
Column: Eclipse XDB-C8 5 µm, 4.6 × 150 µm
Mobile phase A: 50 mM ammonium acetate
Mobile phase B: Acetonitrile
Gradient conditions: Mobile phase B% (min) 0(0)-20(5)-85(10)-100(15)
Flow rate: 1 mL/min
Wavelength: 280 nm

### [Comparative Example 1: Production of ethylene glycol salicylate, acid catalyst: sulfuric acid]

To a test tube of ChemiStation [EYELA], 5 g (0.036 mol) of salicylic acid, 7.78 g (1.40VR, 0.125 mol) of ethylene glycol, 0.296 g (0.16VR, 0.003 mol) of 98% sulfuric acid were added and heated to an inner temperature of 115°C. The components were stirred for three hours at an inner temperature of 115°C and then stirred for nine hours at an inner temperature of 107°C.

As a result of analyzing the obtained liquid reaction product by an analysis method 1, the yields were SA: 7.3%, SE: 77.3%, SEE: 11.2%, and DSE: 4.3%, the conversion rate was 92.7%, and the selectivity was 83.3%. These results are shown in Table 2.

### [Example 1: Production of ethylene glycol salicylate, solid acid catalyst: solid acid catalyst 1 (DIAION (registered trademark) SK104H)]

A stainless steel column tube (Tokyo Rikakikai Co., Ltd., inner diameter: 10 mm, length: 100 mm) was filled with 5.86 g of a solid acid catalyst 1 [Mitsubishi Chemical Corporation, trade name: DIAION (registered trademark) SK104H, degree of crosslinking: 4%, average particle diameter: 730 µm] (voids in the column were as shown in Table 2) and mounted in a column-type flow reactor (EYELA, MCR-1000 type).

Five grams (0.036 mol) of salicylic acid and 32.3 g (5.8VR, 0.52 mol) of ethylene glycol were added to a 100 mL dissolution tank and dissolved at room temperature. The obtained solution was suctioned with a 10 mL syringe pump and delivered to the column-type flow reactor for which the flow rate was set to 0.039 mL/min (residence time: 60 min) and the column temperature was set to 105°C, and a reaction was initiated.

As a result of analyzing the obtained liquid reaction product by the analysis method 1, the yields were SA: 11.2%, SE: 82.7%, SEE: 5.5%, and DSE: 0.7%, the conversion rate was 88.8%, and the selectivity was 93.1%. These results are shown in Table 2.

### [Example 2: Production of ethylene glycol salicylate, solid acid catalyst: solid acid catalyst 3 (DIAION (registered trademark) UBK530K)]

A reaction was performed in the same manner except that, in Example 1, 5.86 g of the solid acid catalyst 1 was changed to 5.97 g of a solid acid catalyst 3 [Mitsubishi Chemical Corporation, trade name: DIAION (registered trademark) UBK530K, average particle diameter: 360 µm] (voids in the column were as shown in Table 2), the flow rate was changed from 0.039 mL/min (residence time: 60 min) to 0.042 mL/min (residence time: 60 min), and the column set temperature was changed from 105°C to 110°C.

As a result of analyzing the obtained liquid reaction product by the analysis method 1, the yields were SA: 7.2%, SE: 82.3%, SEE: 10.2%, and DES: 0.3%, the conversion rate was 92.8%, and the selectivity was 88.7%. These results are shown in Table 2.

### [Example 3: Production of ethylene glycol salicylate, solid acid catalyst: solid acid catalyst 5 (Amberlite (registered trademark) XH2071H)]

A reaction was performed in the same manner except that, in Example 1, 5.86 g of the solid acid catalyst 1 was changed to 5.30 g of a solid acid catalyst 5 [Organo Corporation, trade name: Amberlite (registered trademark) XH2071H] (voids in the column were as shown in Table 2), and the flow rate was changed from 0.039 mL/min (residence time: 60 min) to 0.036 mL/min (residence time: 90 min).

As a result of analyzing the obtained liquid reaction product by the analysis method 1, the yields were SA: 13.2%, SE: 81.2%, SEE: 4.8%, and DSE: 0.5%, the conversion rate was 86.8%, and the selectivity was 93.5%. These results are shown in Table 2.

### [Example 4: Production of ethylene glycol salicylate, solid acid catalyst: solid acid catalyst 6 (TAYCACURE (registered trademark) SAC-6)]

A reaction was performed in the same manner except that, in Example 1, 5.86 g of the solid acid catalyst 1 was changed to 5.0 g of a solid acid catalyst 6 [Tayca Corporation, trade name: TAYCACURE (registered trademark) SAC-6, average particle diameter: 200 µm, specific surface area: 450 m²/g] (voids in the column were as shown in Table 2), the flow rate was changed from 0.039 mL/min (residence time: 60 min) to 0.049 mL/min (residence time: 90 min), and the column set temperature was changed from 105°C to 130°C.

As a result of analyzing the obtained liquid reaction product by the analysis method 1, the yields were SA: 10.9%, SE: 81.0%, SEE: 7.7%, and DSE: 0.4%, the conversion rate was 89.1%, and the selectivity was 90.9%. These results are shown in Table 2.

In Table 2 and Table 3, which will be shown below, "Productivity" represents the yield of a target that is obtained per unit time and is a value obtained by dividing the yield of the target by the reaction time.

**[Table 2]**

| | Kind of acid catalyst | Void **[mL]** | Reaction method | Reaction temperature **[°C]** | Reaction time **[min]** | Flow rate **[mL/min]** | Yield **[%]** | | | | Conversion rate **[%]** | Selectivity **[%]** | Productivity **[%/min]** |
|---|---|---|---|---|---|---|---|---|---|---|---|---|---|
| | | | | | | | **SA** | **SE** | **SEE** | **DSE** | | | |
| Example **1** | Solid acid catalyst **1** | **2.36** | Flow | **105** | **60** | **0.039** | **11.2** | **82.7** | **5.5** | **0.7** | **88.8** | **93.1** | **1.38** |
| Example **2** | Solid acid **catalyst3** | **2.52** | Flow | **110** | **60** | **0.042** | **7.2** | **82.3** | **10.2** | **0.3** | **92.8** | **88.7** | **1.37** |
| Example **3** | Solid acid catalyst **5** | **3.23** | Flow | **105** | **90** | **0.036** | **13.2** | **81.2** | **4.8** | **0.5** | **86.8** | **93.5** | **0.90** |
| Example **4** | Solid acid catalyst**6** | **4.45** | Flow | **130** | **90** | **0.049** | **10.9** | **81.0** | **7.7** | **0.4** | **89.1** | **90.9** | **0.90** |
| Comparative Example 1 | Sulfuric acid | - | Batch | **107-115** | **720** | - | **7.3** | **77.3** | **11.2** | **4.3** | **92.7** | **83.3** | **0.11** |

As is clear from Examples 1 to 4 and Comparative Example 1 in Table 2, when a solid acid catalyst is used, and the flow method is employed as the reaction method, no waste acid treatment is required, and ethylene glycol salicylate can be produced at a high productivity while the generation of an impurity is suppressed.

### [Comparative Example 2: Production of methyl salicylate, acid catalyst: sulfuric acid]

To a test tube of ChemiStation [EYELA], 5 g (0.036 mol) of salicylic acid, 10.2 g (2.57VR, 0.317 mol) of methanol, 0.353 g (0.0036 mol) of 98% sulfuric acid were added, heated to a jacket temperature of 115°C (reflux) and stirred for four hours. As a result of analyzing the obtained liquid reaction product by the analysis method 1, the yields were SA: 60.8%, SM: 39.2%. These results are shown in Table 3.

### [Example 5: Production of methyl salicylate, solid acid catalyst: solid acid catalyst 1 (DIAION (registered trademark) SK104H)]

A stainless steel column tube (Tokyo Rikakikai Co., Ltd., inner diameter: 10 mm, length: 100 mm) was filled with 5.86 g of the solid acid catalyst 1 [Mitsubishi Chemical Corporation, trade name: DIAION (registered trademark) SK104H] (voids in the column were as shown in Table 3) and mounted in the column-type flow reactor (EYELA, MCR-1000 type).

Five grams (0.036 mol) of salicylic acid and 19.8 g (5VR, 0.616 mol) of methanol were added to a 100 mL dissolution tank and dissolved at room temperature. The obtained solution was suctioned with the 10 mL syringe pump and delivered to the column-type flow reactor for which the flow rate was set to 0.305 mL/min (residence time: 10 min) and the column temperature was set to 120°C, and a reaction was initiated.

As a result of analyzing the obtained liquid reaction product by the analysis method 1, the yields were SA: 12.7% and SM: 87.3%. These results are shown in Table 3.

### [Example 6: Production of methyl salicylate, solid acid catalyst: solid acid catalyst 2 (DIAION (registered trademark) SK1BH)]

A reaction was performed in the same manner except that, in Example 5, 5.86 g of the solid acid catalyst 1 was changed to 5.85 g of a solid acid catalyst 2 [Mitsubishi Chemical Corporation, trade name: DIAION (registered trademark) SK1BH] (voids in the column were as shown in Table 3) and the flow rate was changed from 0.305 mL/min (residence time: 10 min) to 0.155 mL/min (residence time: 20 min).

As a result of analyzing the obtained liquid reaction product by the analysis method 1, the yields were SA: 6.9% and SM: 93.1%. These results are shown in Table 3.

**[Table 3]**

| | Kind of acid catalyst | Void **[mL]** | Reaction method | Reaction temperature **[°C]** | Reaction time **[min]** | Flow rate **[mL/min]** | Yield **[%]** | | Productivity **[%/min]** |
|---|---|---|---|---|---|---|---|---|---|
| | | | | | | | **SA** | **SM** | |
| Example **5** | Solid acid catalyst**1** | **3.05** | Flow | **120** | **10** | **0.305** | **12.7** | **87.3** | **8.73** |
| Example **6** | Solid acid catalyst**2** | **3.16** | Flow | **120** | **20** | **0.155** | **6.9** | **93.1** | **4.66** |
| Comparative Example **2** | Sulfuric acid | - | Batch | **115** | **240** | - | **60.8** | **39.2** | **0.16** |

As is clear from Examples 5 and 6 and Comparative Example 2 in Table 3, when a solid acid catalyst is used, and the flow method is employed as the reaction method, no waste acid treatment is required, and methyl salicylate can be produced at a high productivity.

### [Industrial Applicability]

The method for producing a salicylic acid ester of the present invention is a safe method for producing a salicylic acid ester, in which a salicylic acid ester can be produced within a short reaction time at a high selectivity, a high yield and a high productivity, furthermore, the environmental load is low, and the amount of an impurity generated is small and also a production method enabling the continuous production of a salicylic acid ester and is thus industrially useful.

The present invention has been described in detail using a specific aspect, but it is obvious to persons skilled in the art that the present invention can be modified in a variety of manner within the intention and scope thereof.

The present application claims priority based on Japanese Patent Application No. 2022-047108, filed March 23, 2022, and the entire content thereof is incorporated herein by reference.

### [Reference Signs List]

- 1: Preparation tank
- 2: Solid acid catalyst
- 3: Reaction container
- 4: Collection tank
- 5: Back pressure valve

## Claims

1. A method for producing a salicylic acid ester represented by general formula (2) below, wherein salicylic acid and an alcohol represented by general formula (1) below are brought into contact with a solid acid catalyst at a reaction temperature of 50°C to 200°C:
X-R-OH ... (1)
(in the formula (1), R represents a linear or branched alkyl group having 1 to 6 carbon atoms. X represents a hydrogen atom or a hydroxyl group), 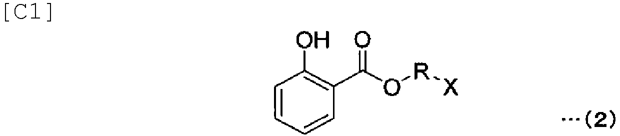 (in the formula (2), R and X are each as defined in the formula (1)).

2. The method for producing a salicylic acid ester according to claim 1, wherein the contact method is a method in which the salicylic acid and the alcohol are passed through a column filled with the solid acid catalyst.

3. The method for producing a salicylic acid ester according to claim 1 or 2, wherein the solid acid catalyst contains an organic adsorbent or an inorganic adsorbent as a base and has a strong acidic ion exchange group.

4. The method for producing a salicylic acid ester according to any one of claims 1 to 3, wherein the solid acid catalyst has an average particle diameter of 1 µm to 2000 µm.

5. The method for producing a salicylic acid ester according to any one of claims 1 to 4, wherein the solid acid catalyst has a specific surface area of 200 m²/g to 2000 m²/g.

6. The method for producing a salicylic acid ester according to any one of claims 1 to 5, wherein the reaction temperature is 70°C to 180°C.
